# EUROPEAN PATENT APPLICATION

(11) **EP 1 779 933 A1**
(43) Date of publication of application: **02.05.2007**
(21) Application number: 05023369.1
(22) Date of filing: 26.10.2005
(51) Int. Cl.: B05B 11/00, B65D 83/00, B05B 11/04, A61M 11/00, B65D 1/02, B29C 65/74, B65D 6/00

(54) **Dispenser for a liquid**

(71) Applicant: The Procter and Gamble Company, Cincinnati, Ohio 45202 (US)
(72) Inventor: Stark, Roland, Bracknell Berkshire RG42 3TQ (GB); Jarvis, Mark Andrew, Berkshire SL6 2EJ (GB)
(74) Representative: Clemo, Nicholas Graham

(57) **Abstract**

A dispenser (1) for a liquid is provided which is particularly, though not exclusively, useful for dispensing liquid medical compositions such as nasal sprays. The dispenser employs at least one, though preferably two, semi-rigid casing halves (2 and 3), defining a liquid reservoir (12), within which is mounted a bridge (21) member which performs the functions of dip tube, dose metering pump, and outlet orifice. An air inlet (8) may be provided into the reservoir, to allow air to enter as liquid leaves, in which case it may be provided with a liquid-impermeable, and preferably bacteria-impermeable, cover (9). Other constructions are described in which no such cover need be provided, or in which the need for an air inlet can be avoided altogether.

## Description

This invention relates to a dispenser for a liquid, and preferably to a device for dispensing liquid in the form of a spray. The dispenser of the present invention can be used, for example, for dispensing liquids such as medicines, including nasal and throat sprays, compositions for application to the eyes and ears, for example eye drops, eardrops and ear sprays, perfumes, cosmetics, cleaning products and the like. In the following description particular reference will be made to the use of the dispenser in relation to medicines, though it is to be understood that the dispenser has many uses, including those just mentioned above.

Particularly in the field of dispensers for medicines, there are a significant number of challenges which have to be met in order for a dispenser to be properly effective. These include:
(a) the need to maintain the sterility of the contents of the dispenser throughout its life.
(b) the ability to dispense a dose to the degree of accuracy appropriate to the use concerned, and to do so not just when the device is first used but throughout all subsequent uses.
(c) the avoidance of clogging at the dispenser outlet (this is not only advantageous for its own sake, but is also relevant to the achievement of both (a) and (b) just mentioned).
(d) ease and cheapness of manufacture, at least to the extent permitted by the need to achieve the preceding aims.
(e) compactness, at least in the case of dispensers which the user may need to carry around for use during the day.

There are numerous proposals in the art for dispensers which are intended to meet at least some of the preceding aims, and, by way of example, attention is directed to WO03/097250 (Genosar), US Patent 6,460,781 (Garcia et al.) and US Patent 6,769,579 (Milian et al.). However, although benefits may be achievable by use of dispensers made in accordance with these and other proposals, none is believed to be wholly satisfactory in all respects, and the present invention is directed, in its various aspects, to the achievement of some or all of the above aims, and to the achievement of other aims which will become apparent from the ensuing description.

According to a first aspect of the invention, there is provided a dispenser for a respiratory tract treatment composition, comprising:
a) a reservoir containing a fluid which is a respiratory tract treatment composition; and
b) a pump in fluid communication with the reservoir, the pump comprising a dose chamber and a pump actuator; and
c) an outlet orifice in fluid communication with the reservoir and the pump such that actuation of the pump is able to effect delivery of fluid from the dispenser through the outlet orifice,
characterised in that the reservoir comprises a laminated container which is adapted to delaminate as fluid is dispensed therefrom.

In a further aspect the invention provides a dispenser for a liquid comprising:
a) a reservoir suitable for containing the liquid, the reservoir comprising a laminated container comprising an outer layer which comprises a rigid material and at least one further layer inwardly thereof, which is delaminatable from the outer layer, and
b) a pump in fluid communication with the reservoir and with an outlet orifice, the pump comprising an upper pump half comprising a resilient actuator connected to the upper pump half, and a lower pump half, wherein depression of the resilient actuator is effective to dispense a dose of fluid via the pump, and wherein the upper and lower pump halves are each formed from unitary mouldings.

In another aspect the invention provides a liquid dispenser comprising
a) a pump body including two spaced-apart major surfaces having interconnected peripheral edges to define therebetween a substantially closed reservoir to contain a liquid to be dispensed therefrom, the major part of said body being substantially rigid,
b) a pumping chamber having an inlet valve communicating with said reservoir, and an outlet valve communicating with an outlet orifice, the chamber being actuated by a pumping button provided along one of said major surfaces, wherein said pumping button defines a part of one of said major surfaces and serves as a wall of said pumping chamber and depression of the pumping button to its displaced position is adapted to cause liquid contained in said pumping chamber to be expelled via said outlet valve, and return of said button to its first normal position causes liquid to enter said pumping chamber via said inlet valve; wherein the dispenser further comprises an air inlet opening, in fluid communication with the reservoir, enabling ambient air to enter the reservoir during return of said button to its first normal position and following dispensing of the fluid, the air inlet opening being occluded by a cover which is gas-permeable, to permit gas to flow into the reservoir, but impermeable to the liquid contained within the reservoir preferably the gas-permeable cover is impervious to microorganisms which might otherwise enter the reservoir.

In yet another aspect the invention provides a liquid dispenser comprising
a) a body including two spaced-apart major surfaces having interconnected peripheral edges to delimit there between a closed reservoir capable of containing a liquid to be dispensed, the major part of said body being substantially rigid;
b) a pump actuated by a pumping button provided along one of said major surfaces, wherein said pumping button is formed as a part of one of said major surfaces; wherein the dispenser further comprises a dispensing nozzle in fluid communication with the pump and comprising an outlet orifice for dispensing the fluid, said nozzle being provided with an expulsion channel and with a closure member disposed in fixed manner in the expulsion channel upstream from said outlet orifice, said closure member comprising a fixed portion fixed relative to the outlet orifice and an elastically-deformable portion supporting a closure element which, at rest, closes off the outlet orifice and which, when the elastically-deformable portion deforms, opens the outlet orifice.

In a preferred form of the aspect first described, the pump comprises a pumping chamber which has an inlet valve and is actuated by said pumping button, which is provided along one of said major surfaces, wherein said pumping button is formed as a part of one of said major surfaces and serves as a wall of said pumping chamber, and depression of the pumping button to its displaced position causes liquid contained in said pumping chamber to be expelled and return of said button to its first normal position causes liquid to enter said pumping chamber via said inlet valve, the dispensing nozzle being in fluid communication with the pumping chamber.

In any of its aspects, both those described above, and those described below, the dispenser according to the invention preferably comprises a cover that engages with the body of the dispenser so as to cover and protect at least the outlet orifice from the external environment, the cover comprising a closure element that, when the cover is fitted in the closed position, sealing engages with the outlet orifice and/or with a surface surrounding the outlet orifice.

In a further aspect, the invention further provides a pump for dispensing a fluid from a reservoir, comprising a pump chamber comprising a bottom plate, inlet and outlet ports, an actuator that forms at least part of at least one of the pump chamber walls, and a deformable resistance device within the pump chamber, interposed between the bottom plate and the actuator, such that the deformable resistance device does not interfere significantly with the initial deformation of the actuator during actuation but resists the final deformation of the actuator, the deformation of the deformable resistance device not occurring until a threshold force is overcome, thereby providing a feedback signal to the user in order to determine complete actuation.

In a yet further aspect the invention provides a fluid dispenser comprising a reservoir containing the fluid substance to be dispensed, and an outlet orifice, said reservoir including at least one actuating wall or wall portion that can be deformed by applying a pressing force so as to reduce the internal volume of the reservoir and thus exert pressure on the fluid substance so as to deliver it through the outlet orifice, said at least one actuating wall having a predetermined threshold of resistance to deformation that must be overcome in order to deform it; wherein the predetermined threshold of resistance to deformation of the actuating wall is generated by threshold resistance means positioned immediately under said actuating wall or wall portion, said threshold resistance means exerting substantially no pressure on the fluid contained within the reservoir once the predetermined threshold of resistance to deformation is overcome.

In any of its aspects, the outlet of the dispenser according to the invention preferably defines a fluid delivery axis, and the pump is actuated by a force directly applied to the dispenser at an angle of at least 60° to the fluid delivery axis.

In another aspect, the invention provides a fluid dispenser comprising:
a) a body comprising an upper half comprising a major surface and a lower half comprising a major surface, the major part of said body being substantially rigid, the two major surfaces being opposite one another and having peripheral edges which are interconnected to define therebetween a substantially closed reservoir for containing a liquid to be dispensed therein, the peripheral edges of the two halves comprising connection means to enable fluid tight sealing of the two halves, wherein the peripheral edges of the two halves define a fluid outlet opening when interconnected; and
b) a bridge piece defining at least part of a fluid inlet channel and interposed between the upper and lower halves, the bridge piece further comprising a fluid outlet channel body that emerges from the fluid dispenser via the fluid outlet opening defined by the upper and lower halves, the fluid outlet channel body comprising sealing means that engages the edges of the fluid outlet opening in a fluid-tight seal.

In yet another aspect, the invention provides a fluid dispenser comprising:
a) a body comprising an upper half comprising a major surface and a lower half comprising a major surface, the major part of said body being substantially rigid, the two major surfaces being opposite one another and having peripheral edges which are interconnected to define therebetween a substantially closed reservoir for containing a liquid to be dispensed therein, the peripheral edges of the two halves comprising connection means to enable fluid tight sealing of the two halves, wherein the peripheral edges of the two halves define a fluid outlet opening when interconnected, wherein one of the said halves is formed from a material which is substantially absorbent to laser radiation of a wavelength capable of welding the two halves together, and the other of the said halves is substantially transmissive to the said radiation; and
b) a bridge piece comprising pumping means and defining at least part of a fluid dispensing channel that sits between the upper and lower halves, the bridge piece further defining a fluid outlet channel body that emerges from the fluid dispenser via the fluid outlet opening defined by the upper and lower halves.

In a yet further aspect, the invention provides a fluid dispenser comprising:
a) an upper dispenser half comprising a major surface, the major part of said upper dispenser half being substantially rigid, the peripheral edges of said upper half extending in a generally perpendicular direction from the plane of the major surface, said upper half further comprising a pumping button formed as a part of said major surface and serving as a wall of a pumping chamber;
b) a bridge piece comprising a pump chamber bottom plate, said pump chamber bottom plate comprising an inlet port and an outlet port in fluid communication with the pumping chamber, wherein the pump chamber bottom plate sealingly engages with the pumping button of the upper dispenser half so as to form a sealed pumping chamber;
c) an inlet valve in fluid communication with the inlet port;
d) an outlet valve in fluid communication with the outlet port; and
e) a lower dispenser half comprising a flexible material sealingly bonded to the perpendicular peripheral edges of the rigid upper dispenser half so as to define a flexible reservoir wall and such that the return of the pumping button following dispensing causes fluid to be taken up into the pumping chamber via the inlet valve and inlet port generating a reduced pressure in the reservoir resulting in deformation of the flexible lower dispenser half to adapt to the reduced volume therein.

In another aspect, the invention provides a bridge piece for a modular fluid dispenser and pump, said bridge piece being of unitary construction and comprising:
a) an inlet channel wall defining at least part of an inlet fluid channel;
b) a pump chamber bottom surface defining at least one wall of a pump chamber;
c) an inlet port communicating the inlet fluid channel with the pump chamber;
d) an outlet port in fluid communication with the pump chamber; and
e) a fluid outlet channel body in fluid communication with the outlet port, said fluid outlet channel body defining a fluid outlet channel therein.

As already indicated, a dispenser according to the present invention may be used, and in some aspects of the invention, is used, to dispense a fluid composition comprising a medicament, for example to dispense compositions for treatment of the human respiratory tract. These will now be described further by way of example.

### Respiratory Tract Treatment Compositions

The fluid is preferably a pharmaceutically acceptable composition comprising a pharmaceutically acceptable treatment agent selected from medicaments, flavours, salts, surfactants and mixtures thereof. The fluid optionally comprises other adjuvants dissolved or dispersed within it. The fluid can be aqueous or non-aqueous. Suitable aqueous fluids include water and mixtures of water with water-miscible solvents such as glycerol, propylene glycol, or alcohols such as ethanol or isopropyl alcohol. Aqueous emulsions can also be used, either water-in-oil or oil-in water emulsions. Preferably the fluid is an aqueous solution, dispersion or oil-in-water emulsion. Suitable non-aqueous fluids comprise polyethylene glycols, glycerol, propylene glycol, dimethyl isosorbide, silicone oils, ketones, ethers and mixtures thereof.

In preferred embodiments the fluid is a pharmaceutically acceptable intranasal carrier and preferably isotonic, i.e., it has the same osmotic pressure as blood and lacrimal fluid. The desired isotonicity of the fluid composition can be accomplished using, for example, sodium chloride or other pharmaceutically-acceptable agents such as dextrose, boric acid, citric acid, sodium tartrate, sodium citrate, sodium phosphate, potassium phosphate, propylene glycol or other inorganic or organic solutes. Sodium chloride is preferred particularly for buffers containing sodium ions. Further examples of sodium chloride equivalents are disclosed in Remington's Pharmaceutical Sciences pp. 1491-1497 (Alfonso Gennaro 18th ed. 1990).

The fluid herein, which is preferably a nasal treatment composition, preferably has a viscosity of less than about 1,000 mPas but generally at least 10 mPa.s. More preferred viscosity values are from 20 to about 500 mPa.s and from about 50 to 500 mPa.s.

### Medicaments

The fluid composition can comprise a wide range of medicaments. By "medicament" is meant a drug or other substance intended to have a therapeutic effect on the body. Suitable levels of the medicament are from 0.001 to 20%, preferably from 0.01 to 5%, more preferably from 0-1 to 5%. It will be appreciated that the levels of specific medicaments will depend on many factors including their potency, safety profile, solubility / ease of dispersion and intended effect. The medicament, when used, can be one which is intended to have an effect at the site of application, such as a decongestant, antihistamine, analgesic or anti-inflammatory drug, or it may be intended for systemic absorption such as an anti-viral, anti-depressant, anti-emetic, anti-pyretic medicament or a hormone or such-like. The medicament can be soluble in the fluid or can be an insoluble, finely divided particulate liquid or solid dispersed within the fluid.

Suitable decongestants include oxymetazoline, tramazoline, xylometazoline, naphazoline, tetrahydrazoline, pseudoephedrine, ephedrine, phenylephrine, their pharmaceutically acceptable salts, such as the hydrochlorides, and mixtures thereof. Preferred decongestants are selected from oxymetazoline, xylometazoline, their pharmaceutically acceptable salts and mixtures thereof. Suitably, a decongestant is present at a concentration of from about 0.01% to about 3.0%, more preferably from about 0.01% to about 1%.

Useful antihistamines include fast-acting, histamine H-1 receptor antagonists. Such H-1 receptor antihistamines may be selected from among the following groups of antihistamines: alkylamines, ethanolamines, ethylenediamines, piperazines, phenothiazines, piperidines. Examples of useful fast acting antihistamines include acrivastine, carbinoxamine, diphenhydramine, chloropheniramine, brompheniramine, dexchloropheniramine, doxylamine, clemastine, promethazine, trimeprazine, methdilazine, hydroxyzine, pyrilamine, tripelennamine, meclizine, triprolidine, azatadine, cyproheptadine, rocastine, phenindamine or pharmaceutically acceptable salts and mixtures thereof. Other useful antihistamines include terfenadine, azelastine, cetirizine, astemizole, ebastine, ketotifen, lodoxamide, loratadine, levocabastine, mequitazine, oxatomide, setastine, tazifylline, temelastine or pharmaceutically acceptable salts and mixtures thereof. When used, the antihistamine component is preferably present in the nasal treatment fluid at a concentration of from about 0.01 % to about 3.0%, more preferably from about 0.01 % to about 1%.

The medicament can also be an anti-inflammatory agent such as a corticosteroid. Particularly preferred agents within this class are glucocorticoids selected from the group consisting of beclomethasone, flunisolide, fluticasone, memetasone, budesonide, pharmaceutically acceptable salts thereof and mixtures thereof. An anti-inflammatory agent is preferably present in the fluid at a concentration of from about 0.001% to about 0.1%, more preferably from about 0.01% to about 0.1%.

Also useful herein are xanthine derivatives such as caffeine and methyl-xanthine and the like; antiallergics; mucolytics; anticholinergics; non-opiate analgesics such as acetaminophen, acetylsalicylic acid, ibuprofen, etodolac, fenbuprofen, fenoprofen, ketorolac, flurbiprofen, indomethacin, ketoprofen, naproxen, pharmaceutically acceptable salts thereof and mixtures thereof; opiate analgesics such as butorphanol; leukotriene receptor antagonists; mast cell stabilisers such as cromolyn sodium, nedocromil and lodoxamide; and lipoxygenase inhibiting compounds.

Further examples of suitable medicaments can be found in WO97/46243, EP-A-780127, US-A-5,124,315, US-A-5,622,724, US-A-5,656,255 and US-A-5,705,490

### Flavours

Various flavouring and/or aromatic components (e.g., aldehydes and esters) can be used in the present nasal treatment fluids. These include, for example, menthol, camphor, eucalyptol, benzaldehyde (cherry, almond); citral (lemon, lime); neral; decanal (orange, lemon); aldehyde C-8, aldehyde C-9 and aldehyde C-12 (citrus fruits); tolyl aldehyde (cherry, almond); 2,6-dimethyl-octanal (green fruit); 2-dodecenal (citrus, mandarin); and herbal components such as thyme, rosemary and sage oils. Additional aromatic components suitable for use in the present invention include those described in U.S. Patent 4,136,163 to Watson et al., U.S. Patent 4,459,425 to Amano et al., and U.S. Patent 4,230,688 to Rowsell et al. Mixtures of these aromatics can also be used.

### Surfactants

The treatment fluid can also comprise one or more pharmaceutically acceptable surfactants. Such surfactants can be useful for dispersing or emulsifying medicaments or flavours, for enhancing absorption across the nasal or mucous membranes or as treatment agents in their own right The surfactants can be anionic, nonionic, cationic or amphoteric, preferably they are nonionic. Typical nonionic surfactants useful herein include: polyoxyethylene derivatives of fatty acid partial esters of sorbitol anhydrides such as polysorbate 80; polyoxyethylene derivatives of fatty acids such as polyoxyethylene 50 stearate, as well as oxyethylated tertiary octyl phenol formaldehyde polymer (available from Sterling Organics as Tyloxapol) or mixtures thereof. The usual concentration of surfactant is from about 0.1% to about 3% by weight.

### Salts

The fluid can also comprise one or more pharmaceutically acceptable salts-The salt can mineral salts such as e.g. sodium chloride, or salts of organic acids such as sodium citrate.

### Other adjuvants

The fluid herein can further comprise other ingredients such as thickeners, humectants, suspending aids, encapsulating aids, chelating agents and preservatives.

The viscosity of the compositions may be maintained at a selected level using a phanrrxaceutxcally-acceptable thickening agent. Suitable thickening agents include, for example, xanthan gum, methyl cellulose, microcrystalline cellulose, carboxymethyl cellulose, chitosan, hydroxypropyl cellulose, hydroxypropyl methyl cellulose, hydroxymethyl cellulose, hydroxyethyl cellulose, carboxyvinyl polymer, carbomer, and the like or pharmaceutically acceptable salts thereof. Mixtures of such thickening agents may also be used. The preferred concentration of the thickener will depend upon the agent selected. The important point is to use an amount which will achieve the selected viscosity. Viscous compositions are normally prepared from solutions by the addition of such thickening agents.

Fluids useful in the present invention can also comprise from about 0.01 % to about 5% of a humectant to inhibit drying of the mucous membrane and to prevent irritation. Any of a variety of pharmaceutically-acceptable humectants can be employed including, for example sorbitol, propylene glycol, polyethylene glycol, glycerol or mixtures thereof. As with the thickeners, the concentration will vary with the selected agent, although the presence or absence of these agents, or their concentration is not an essential feature of the invention.

A pharmaceutically-acceptable preservative may be employed to increase the shelf life of the compositions herein. A variety of preservatives including, for example, benzyl alcohol, parabens, phenylethyl alcohol, thimerosal, chlorobutanol, chlorhexidine gluconate, or benzalkonium chloride can be employed. A suitable concentration of the preservative will be from 0.001 % to 2% based on the total weight, although there may be appreciable variation depending upon the agent selected. However, the provision, in some aspects of the invention, of an air inlet into the reservoir which is impervious to potentially contaminating micro-organisms and/or the use of a closure member at the outlet orifice which closes off the orifice to the exterior except when a dose is being dispensed, and/or the use of a laminated container, in which air entering the dispenser during use cannot come into contact with the liquid to be dispensed, may render the use of a preservative unnecessary.

### Definitions

Unless specified otherwise, all percentages and ratios herein are by weight and all measurements are made at 25°C. All viscosity measurements herein are made, unless otherwise specified, at 25°C and at a shear rate of 1s⁻¹, using a steady state flow method.

In preferred embodiments the dispensers and pumps herein comprise upper and lower halves. The terms "half" and "halves" herein are intended only to indicate corresponding parts and are not intended to imply any equality in size, construction or function. The terms "upper" and "lower" in respect of the pump or are used only to distinguish the two halves and are not intended to imply any particular orientation of the parts in use.

As used herein a "semirigid" thermoplastic material means a thermoplastic material having a flexural modulus of at least about 300, preferably at least about 500 MPa, measured using ASTM D790.

By a "unitary moulding" is meant a moulding formed in a single piece or completely formed within a single mould. It may comprise only one material but the term "unitary moulding" also comprises workpieces formed from two or more materials in a common moulding operation such as a two-shot injection moulding where different materials are co-injected or sequentially injected into a common mould.

A "fluid" herein refers to a flowable liquid or gel.

"Fluid delivery axis" refers to the line that the most of the fluid is travelling in as it leaves the dispensing outlet, where the fluid is breaking up into a spray this will generally represent the line from the dispensing outlet pointing towards the centre of the spray pattern.

The invention is further described below with reference to the accompanying drawings, in which:
Fig. 1 is a view from one side of an embodiment of the invention;
Fig. 2 is a similar view but with a cap forming part of the embodiment removed, so as to reveal the underlying parts of the device;
Fig. 3 is a perspective view of what can be seen in Fig. 2;
Fig. 4 is a cross-section taken on line IV-IV in Fig 1;
Fig. 5 is a longitudinal section through the embodiment of Fig. 1, with the cap wholly removed;
Fig. 6 is a perspective, exploded view, showing certain components of the device of Fig. 1;
Fig. 7 shows one of the components of Fig. 6 from a direction at 180° to that from which it is shown in Fig. 6;
Fig. 8 is a view taken along arrow "A" in Fig. 7;
Fig. 9 is a longitudinal section, on an enlarged scale, of a modified version of the outlet end of the devices;
Fig. 10 shows a further embodiment of the invention.
Fig. 11 shows the two pieces of Fig. 10 assembled to form a pump.
Fig. 12 is a cross section of a reservoir according to an embodiment of the present invention comprising a rigid outer wall and a delaminating inner layer.
Fig. 13 is an alternate embodiment of the present invention combining the pump of Figure 11 which the reservoir of Figure 12.

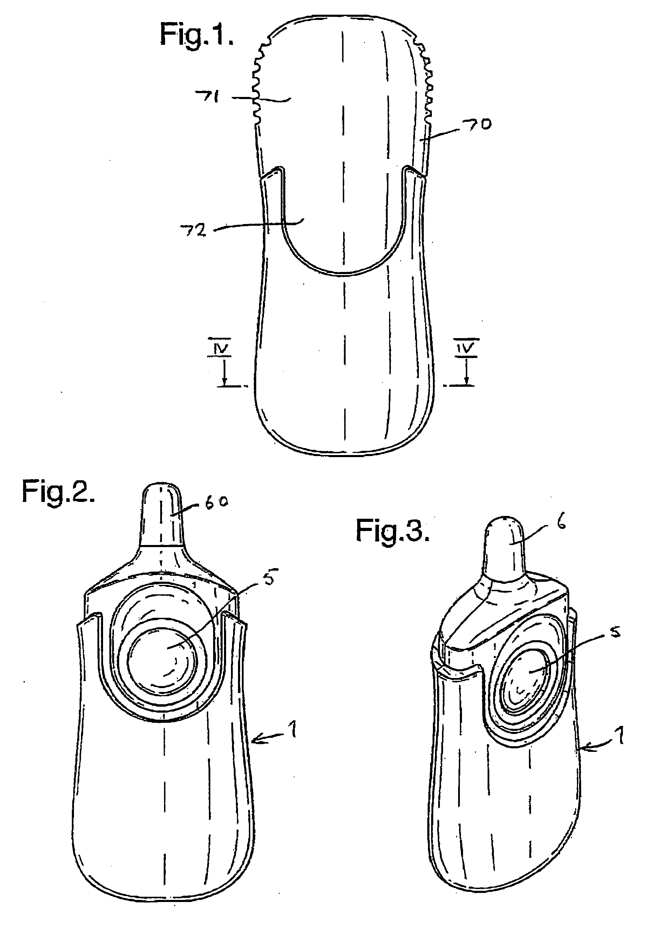

The embodiment shown by way of example in Figs. 1 to 9 is intended as a dispenser for a respiratory tract treatment composition, and, more particular, to such a composition which is administered as a nasal spray. The dispenser 1 comprises a casing formed of two casing parts 2 and 3, which will be referred to for convenience below as the upper half 2 and lower half 3, terms "upper half" and "lower half" being understood as defined above. The casing halves 2 and 3 are of a material which is at least semi-rigid. The casing halves 2 and 3 have respective major surfaces 2a and 3a and peripheral walls 2b and 3b transverse to the major surfaces 2a and 3 a and integral with the peripheral edges of the respective major surfaces. The peripheral walls 2b and 3b terminate in peripheral edges 2c and 3c which are arranged to interlock with one another. In the illustrated embodiment the peripheral edge 2c has a downwardly directed wall 2d, and the peripheral edge 3c has an upwardly directed groove 3d, whereby the wall 2d engages in the groove 3d. It is to be understood, however, that the locations of the wall and groove could be reversed, or that some completely different arrangement could be provided instead.

The upper casing half 2 has, in its major surface, a recessed portion 4 forming a shallow dish, and, within the dish 4 there is formed, a thinner-walled, upwardly domed portion, defining a button 5. The button 5 can be fully integral with the remainder of the upper casing half 2, i.e. formed with the upper casing half and of the same material as the upper casing half, or it can be semi-integral therewith, i.e. coformed therewith (preferably co-moulded) but of a different material to the upper casing half, or it can be a distinct component of the same or a different material. The semi-integral arrangement is preferred, as it can be readily manufactured but permits the use of different materials for the button (preferably a plastics elastomer) and the casing half (preferably a semi-rigxd plastic material). The purpose of this will be explained further below. At the rearward end of the dispenser each casing half has a semi-circular cut out, so that, when the two halves are assembled, they define a circular opening 6. The opening 6 receives a cylindrical plug 7 having a bore 8 within which is received a filter member 9. Although shown as an elongate cylinder, the filter member 9 is preferably a disc of a filter material. The filter 9 is formed of a material whose pore size is such as to permit air to pass therethrough, but so as to prevent the passage of both liquid and bacteria. For this purpose, none of the pores in the filter material must be larger than 0.2 µm in diameter. The filter member 9 is preferably a fibrous material, and, at least where the dispenser is intended to dispense a water-based liquid, the fibers are preferably of a material which is inherently hydrophobic, or are rendered hydrophobic. More preferably the filter member is a membrane formed of PTFE fibers and available under the trade mark GORE-TEX from W. L. Gore Associates, Inc., of Newark, Delaware, USA.

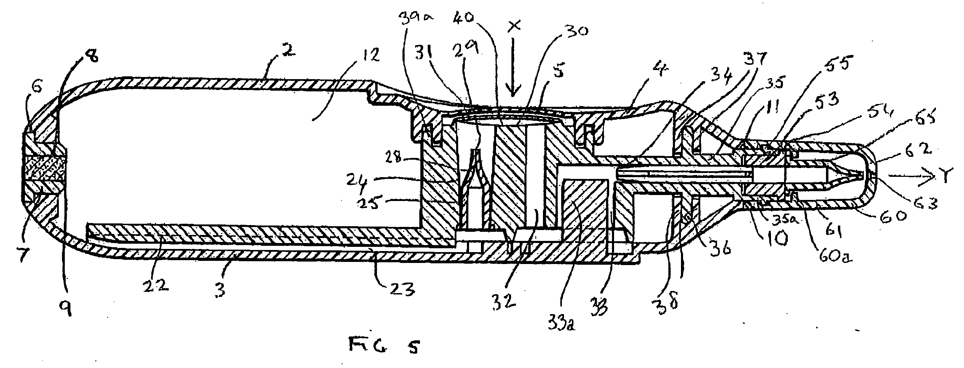

The forward ends of the casing halves 2 and 3 terminate at the plane indicated by reference numeral 10 in Fig. 5, where they define a circular opening 11.

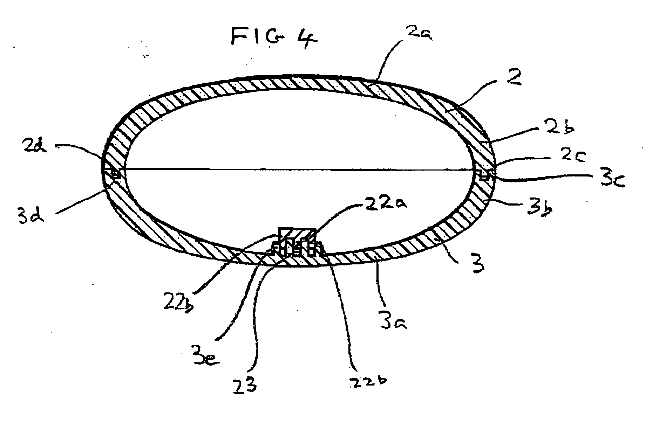

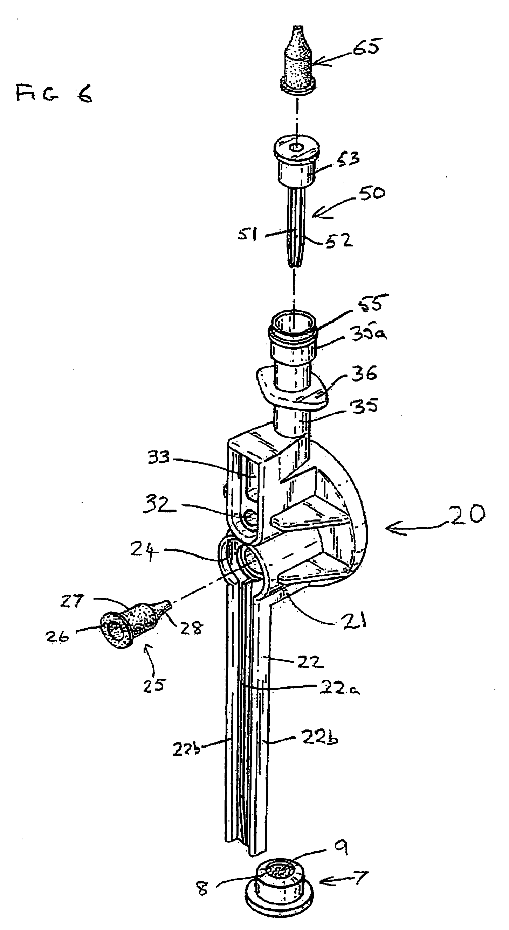

The casing halves 2 and 3 define between them a cavity of which the rear portion 12 forms a reservoir for the liquid to be dispensed. Within the cavity there is mounted a component 20 which serves to permit a metered dose of liquid to be dispensed. The major part of the component 20 is provided by an element 21 which is of unitary construction and which is referred to herein as a bridge. The bridge comprises an elongate channel member 22 which, in the illustrated embodiment, includes a cross-section which defines three sides of a rectangle, the channel member being secured to the lower casing half 3, with the open side of the cross-section facing the channel half 3. As shown in Fig. 4 the channel member 22 has a central rib 22a running parallel to its side walls 22b and the lower casing half 3 has four, mutually parallel ribs 3e. The side walls 22b are a snap fit in the grooves defined by adjacent pairs of ribs 3e (the connection may be assisted by ultrasonic welding, laser welding or an adhesive of a type acceptable in relation to the liquid which the device is intended to contain), The central rib 22a engages between two of the ribs 3e, but so as to leave a space which provides an inlet channel 23. As can be seen in Fig. 6, the upstream portion of the rib 22a preferably reduces progressively in height towards the upstream end. The channel 23 therefore progressively decreases in cross-section from its inlet end. This makes it possible for the channel 23 to have an inlet portion which is of relatively large cross-section, followed by a portion which is of small enough cross-section to exert a capillary effect on the liquid within it.

The downstream end of the channel 23 communicates with a pump chamber 30 via an inlet chamber 24, within which is mounted an inlet valve 25. The inlet valve 25 can be one of a number of types, but in the illustrated embodiment it is formed of a resilient material, for example a synthetic rubber, and comprises a flange 26, a substantially cylindrical body 27, and a pair of surfaces 28 which converge towards one another and which meet in a sealing fashion along a line 29. It is referred to below as duckbill valve 25. The valve 25 acts as a non-return valve, permitting liquid to flow from its flange end to its opposite end and out between the surfaces 28, but preventing flow of fluid in the opposition direction.

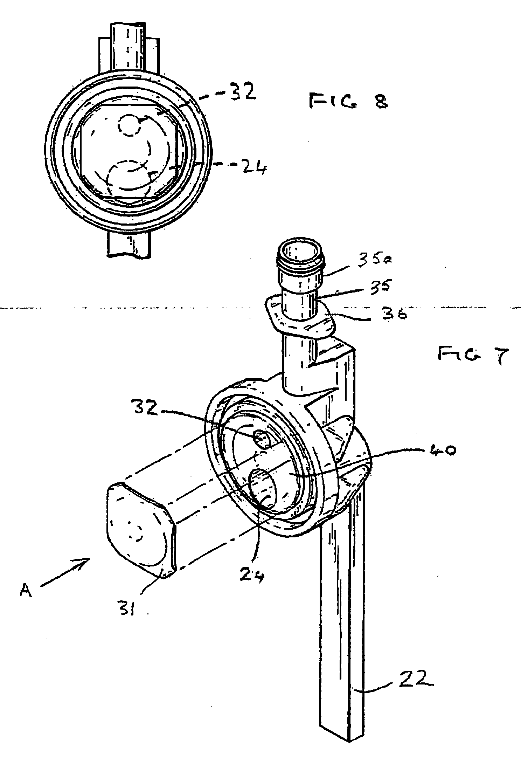

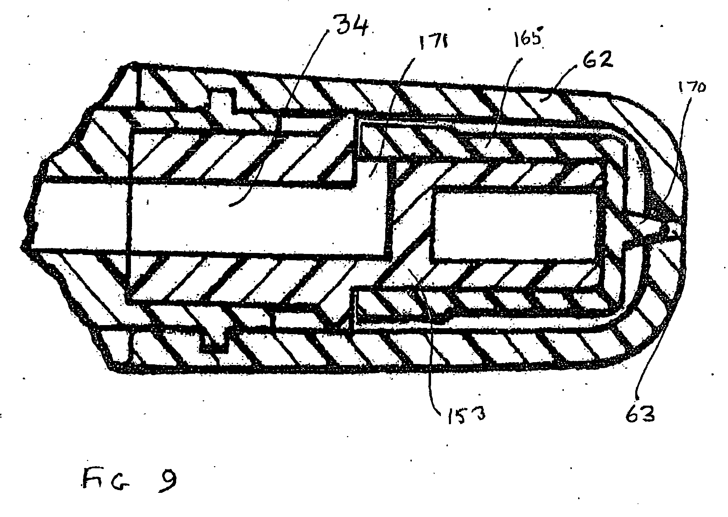

The outlet end of the inlet chamber 24 communicates, as already mentioned, with a pump chamber 30, which is defined on its lower side by a pump chamber bottom surface 40, and which is defined on its upper side by the button 5. An upwardly domed member 31 is seated at its edges on the surface 40, and disposed in parallel with the button 5. The member 31, which, in this embodiment, has the shape of a square with mitred corners (see Figs 7 and 8), allows fluid to pass freely, so that the upper boundary of the chamber is defined by the button 5, not by the member 31. The member 31 can be so arranged as to perform one of two distinct, though interrelated functions. In one arrangement, the member 31 is immediately underneath the button, i.e. the gap shown between the button and the member 31 is negligible. In this arrangement the member is designed to provide a significant resistance to the downward movement of the button preventing movement of the button, until a point is reached whereby a threshold resistance to deformation is surpassed and the button 5 and the member 31 deform suddenly so as to provide rapid reduction in volume in the dose chamber. This has the effect of allowing a build up of significant pressure in the button which is then exerted on the fluid to force it out of the dispenser at a higher velocity. Such a member interposed beneath the button is advantageous as to provide increased threshold force compared with that available via button construction and materials alone. In the other arrangement, there is a significant gap between the button 5 and the member 31, so that for the first part of the travel of the button (the part during which the requite volume of liquid to provide a dose is expelled from the chamber), the member 31 does not interfere significantly with the deformation of the button, and therefore does not resist it. However, a point is then reached in the travel of the button at which the button comes into contact with member (31) and the resistance to the travel of the button increasers. The user must overcome the resistance, providing a feedback signal to the user that a complete dose has been achieved.

The pump chamber 30 communicates with an intermediate chamber 32 which, in turn, communicates with an outlet chamber 33. A volume-reducing element 33a occupies a substantial proportion of the chamber 33, for a purpose which is described below. The outlet chamber 33 communicates with the upstream end of an outlet passage 34 defined by an outlet duct 35. The outlet duct is provided, intermediate its ends, with an outwardly extending flange 36 which is preferably integral with the main portion of the outlet duct 35, and which, as can be seen in Figs. 6 and 7, preferably has a diamond-shape with rounded corners. The flange 36 is received, in a pair of gaps, one defined between two ribs 37 extending downwardly from the upper casing half 2, and the other defined by a pair of ribs 38 extending upwardly from a lower casing half 3, and is sealed therein, for example by ultrasonic spot welding, so forming a sealing means which prevents exit of liquid past the exterior of the outlet duct 35, and entry of air in the opposition direction.

The connection between flange 36 and ribs 37 and 38 forms part of the means by which the bridge is fixedly connected to the casing halves 2 and 3. The remainder of the connection means is provided by the attachment of the channel member 22 to the lower casing half 3, and an annular rib 39a which is upstanding around the pump chamber and which engages between a pair of downwardly directed ribs formed on the underside of the recessed portion 4 and encircling the button 5.

Mounted within the outlet passage 34 is a further volume-reducing element 50. The elements 33a and 50 serve together to reduce the dead space volume between the pump chamber and the outlet of the dispenser. This advantageously reduces the amount of priming needed before the device will expel a dose of liquid through the outlet, in the event that the volume just referred to is empty of liquid before first use. The issue of priming is mentioned below in connection with the operation of the device. In this connection it needs to be kept in mind that a dose of a typical liquid medicament is of the order of 50 µl, so that even a relatively small dead space volume within the dispenser can represent a large number of doses, and therefore a large number of pump chamber volumes This comprises a shaft 51 at its upstream end, the shaft having a plurality of ribs 52 extending radially outwardly. In the illustrated embodiment four such ribs are shown, but it is to be understood that fewer than four, or more than four, could be provided instead. The downstream end portion of the shaft 51 is surrounded by an upstream end portion of a cylindrical member 53, in such a way that the gaps between the downstream ends of the ribs 52 communicate with the interior of the member 53. The duct 35 is provided with an enlarged downstream portion 35a, within which the cylindrical member 53 is a sliding fit. The member 53 has an outwardly extending flange 54 which bears against the downstream end of the portion 35a, to keep the member 50 in its correct longitudinal position. The portion 35a is provided with an outwardly extending flange 55, whose purpose is described below.

The downstream end of the dispenser comprises a cap 60. The cap comprises a generally cylindrical wall 61 and an end wall 62, and an opening 63 which tapers in the downstream direction, is defined in the end wall 62 to provide a spray nozzle. The cylindrical wall 61 is provided, near its upstream end, with an inwardly directed circumferential groove, which is a snap fit over the flange 55, so as to maintain the cap in position. A duckbill valve 65 is secured to the downstream end of the cylindrical member 53 by a flange 60a extending inwardly from the inner surface of the cap 60, so that its interior is in fluid communication with the interior of the member 53. As shown in Fig. 5, the downstream end of the duckbill valve 65 is closely adjacent to the outlet nozzle 63, and this is the operative condition for the dispenser.

The operation of the dispenser will now be described, starting from a condition in which the reservoir 12 is at least partly full of liquid, and the whole of the fluid flow path from the reservoir to the downstream end of the duckbill valve 65 is completely filled with liquid. This will be the condition of the dispenser after the user has employed it to dispense a dose. It may be a condition of the dispenser as initially supplied to the user. However, before first use the pump chamber may require priming with the fluid to be dispensed, to replace air inside the unprimed pump with fluid drawn from the reservoir. The preferred number of priming strokes is three of fewer. The user depresses button 5 by exerting a force on it in the direction of the arrow X. This direction is at 90° to the direction Y in which fluid leaves the dispenser (the fluid delivery axis). Especially in the case of a device for dispensing a nasal or oral spray this is advantageous in terms of ease of use. If, in a modification of the illustrated device, it is not possible, or not convenient, for direction X to be at 90° to direction Y, it should preferably be at an angle to it of at least 60°, more preferably at least 75°. Since the liquid in chamber 24 cannot escape through the duckbill valve 25, depression of the button 5 causes liquid to pass through the intermediate chamber 32, into the passage 34, through the gaps between the ribs 52, through the interior of the cylindrical member 53, through the interior of the duckbill valve 65 and out of the dispenser, as a spray, through the outlet orifice 63. The spray may or may not be an atomised spray, depending on the viscosity of the liquid and the size and shape of the outlet orifice. When the user ceases to exert pressure on the button 5, it returns to its illustrated position, and, in so doing, causes liquid to be sucked into the chamber 24, from the reservoir 12, via the passage 23 and the duckbill valve 25. The device is then available for the dispensing of the next dose, when required.

As shown in Fig. 1, the dispenser is protected against accidental actuation by means of a cover 70. The cover has a main portion 71 which extends over approximately one third of the length of the dispenser, as considered from the outlet end, and further comprises an integral extension 72 which is present only on the side of the dispenser where the button 5 is located. The cover is preferably a snap fit on the underlying parts of the dispenser. The cover 70 may be provided with abutment means on the inside of its upper end for bearing on the upper end of the cap 6. In one form, the abutment means comprises a sealing ring, preferably a resilient material, which bears on the surface of the cup around the outlet orifice 63. In another form, the abutment means comprises a pad of resilient material which bears on the end surface of the cup, including the outlet orifice itself. In both forms, the effect is to isolate the outlet orifice, and therefore liquid upstream of that orifice, from ambient air.

The dispenser shown in Figs. 1 to 8 is preferably assembled using ultrasonic welding or laser welding to join the peripheral edges of the two casing halves to one another. Ultrasonic welding is used conveniently for these purposes, since the same welding apparatus can then be employed as is used to connect the flange 36 to the ribs 37, as mentioned above. When the casing halves are connected by laser welding, one of the two casing halves should be made of a material which is substantially absorbent to laser radiation of a wavelength capable of welding the two halves together, and the other of the casing halves should be made of a material which is substantially transmissive to that radiation. Conveniently thought not essentially, the casing half (in this example the lower casing half) which has the groove 3d is transmissive and the casing half (in this case the upper casing half) which has the wall 2d in absorbent so that welding can be effected by radiation directed at the edge of the casing along lines which are generally horizontal as shown in the view of fig.4.

Many alternative embodiments can be envisaged which also embody some or all of the aspects of the present invention, and some of these will now be described.

Fig. 9. shows a modification in which the duckbill valve 65 is replaced by a hollow sealing member 165 formed of a resilient material, mounted a tubular shaft 153. The sealing member 165 has a conical tip seal 170 formed on the end of the member 165 adjacent the outlet orifice 63. In the position shown in Fig. 9 the tip seal 170 is in sealing engagement with the orifice 63. The tubular shaft 153 has a bore 171 extending through the wall thereof, and in communication with the passage 34. When the user depresses the button 5 the pressure in the passage 34, and therefore in bore 171, rises, causing the part of the member 165 adjacent to the bore to flex outwardly-Fluid is then able to escape past the flexed portion of member 165, and then travel along the gap defined between the member 165 and the cap 62, until it reaches the part of member 165 which carries the tip seal 170. Fluid pressure then causes that part of member 165 to deflect leftwardly (as seen in Fig. 9), bringing the tip seal 170 out of contact with the outlet orifice 63, and thus allow liquid to pass through the orifice. The tip seal 170 prevents exit of liquid and ingress of air when no dose is being dispensed, and helps to prevent clogging of the outlet orifice 63.

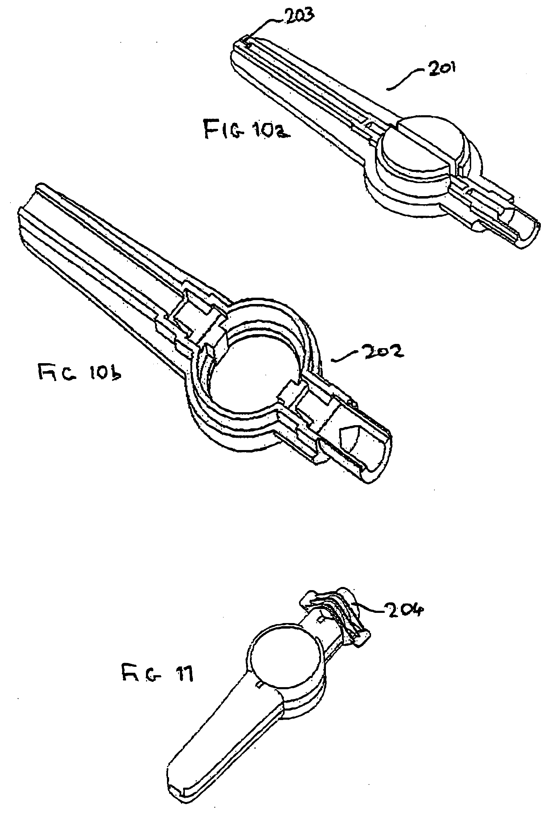

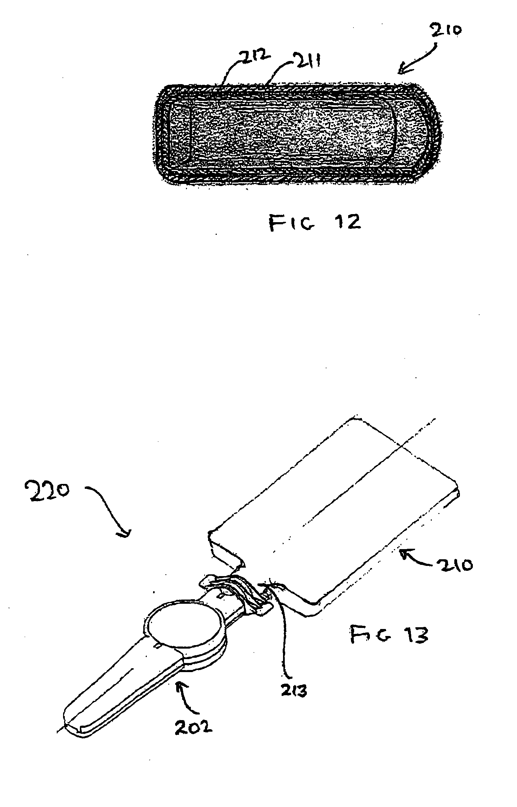

The embodiment shown in Figs 10a to 13 differs from those already described primarily in that the pump unit is exterior to the reservoir. Figs 10a and 10b show diagrammatically two pump halves 201 and 202 each formed as a unitary moulding, the halves being connected together, as shown in Fig 11, to form a complete pump unit. The pump unit has an outlet orifice at one end, which can be seen in part at 203 in Fig 10a. Fig 12 shows a reservoir 210 formed as a laminated container, with an outer layer 211, and an inner layer 212 which, in use, delaminates from the outer layer. The complete dispenser 220, is formed by attaching the outlet 213 of the reservoir 210 to the inlet 204 of the pump unit 202, in fluid communication therewith.

The dispenser described with reference to Figs. 1 to 8 has a casing formed of two casing halves, both of which are at least semi-rigid. However, an alternative possibility is for the major surface in which the button is provided to be made of a semi-rigid material, but the opposite major surface to be made of a flexible material, for example a sheet of a plastics material whose peripheral edges can be attached to edges of the semi-rigid material corresponding to the edges 3c in Fig. 4. Such attachment could, for example, be by means of welding (for example either ultrasonic welding or laser welding), by the use of an adhesive, or in any other suitable fashion. Provided the sheet of flexible material is attached to the semi-rigid component in such a way that the flexible material is not taut, but has a sufficient ability to flex towards the semi-rigid component and thereby reduce the volume between the sheet of flexible material and the semi-rigid component, the air inlet which is provided in the embodiment of Figs. 1 to 8 by the components 6 to 9 can be dispensed with, thus further improving the sterility of the device, and further increasing the possibility of avoiding the need for a preservative in the liquid to be dispensed. In this construction the bridge will need to be attached only to the semi-rigid casing half. Also, instead of forming a dip tube by cooperation of part of the bridge (the channel member 22) and the lower casing half, a conventional dip tube will need to be provided.

In another alternative embodiment, the need for an air inlet could be avoided by employing, for the casing of the dispenser, a so-called laminated construction. Containers are known, though for completely different purposes, in which the container body has an inner layer and an outer layer, and in which a portion of the inner layer delaminates from the outer layer under certain circumstances. By way of example, reference is made to EP-A-0550772 (Yoshino Kogyosho Co., Ltd) for a description of how to form a container in which delamination will occur as a result of the pressure difference which results from the contents of the container being discharged therefrom by the action of a pump. By using a container in which only part of the inner layer delaminates from the outer layer, portions of the inner surface, where delamination does not occur, are available as sites to mount an actuator button and a structure which corresponds to, or at least has a similar function to, the bridge used in the embodiment of Figs. 1 to 8. However, no air inlet is needed, because as delamination occurs, the volume of the reservoir within which the liquid to be dispensed is held can simply reduce of its own accord.

## Claims

1. A dispenser for a respiratory tract treatment composition, comprising:
d) a reservoir containing a fluid which is a respiratory tract treatment composition; and
e) a pump in fluid communication with the reservoir, the pump comprising a dose chamber and a pump actuator; and
f) an outlet orifice in fluid communication with the reservoir and the pump such that actuation of the pump is able to effect delivery of fluid from the dispenser through the outlet orifice,
**characterised in that** the reservoir comprises a laminated container which is adapted to delaminate as fluid is dispensed therefrom.

2. A dispenser for a liquid comprising:
a) a reservoir suitable for containing the liquid, the reservoir comprising a laminated container comprising an outer layer which comprises a rigid material and at least one further layer inwardly thereof, which is delaminatable from the outer layer; and
b) a pump in fluid communication with the reservoir and with an outlet orifice, the pump comprising an upper pump half comprising a resilient actuator connected to the upper pump half, and a lower pump half,
wherein depression of the resilient actuator is effective to dispense a dose of fluid via the pump, and wherein the upper and lower pump halves are each formed from unitary mouldings.

3. A liquid dispenser comprising
a) a pump body including two spaced-apart major surfaces having interconnected peripheral edges to define therebetween a substantially closed reservoir to contain a liquid to be dispensed therefrom, the major part of said body being substantially rigid,
b) a pumping chamber having an inlet valve communicating with said reservoir, and an outlet valve communicating with an outlet orifice, the chamber being actuated by a pumping button provided along one of said major surfaces, wherein said pumping button defines a part of one of said major surfaces and serves as a wall of said pumping chamber and depression of the pumping button to its displaced position is adapted to cause liquid contained in said pumping chamber to be expelled via said outlet valve, and return of said button to its first normal position causes liquid to enter said pumping chamber via said inlet valve;
wherein the dispenser further comprises an air inlet opening, in fluid communication with the reservoir, enabling ambient air to enter the reservoir during return of said button to its first normal position and following dispensing of the fluid, the air inlet opening being occluded by a cover which is gas-permeable, to permit gas to flow into the reservoir, but impermeable to the liquid contained within the reservoir.

4. A dispenser according to claim 3, wherein the gas-permeable cover is impervious to microorganisms which might otherwise enter the reservoir.

5. A liquid dispenser comprising
a) a body including two spaced-apart major surfaces having interconnected peripheral edges to delimit there between a closed reservoir capable of containing a liquid to be dispensed, the major part of said body being substantially rigid;
b) a pump actuated by a pumping button provided along one of said maj or surfaces, wherein said pumping button is formed as a part of one of said major surfaces;
wherein the dispenser further comprises a dispensing nozzle in fluid communication with the pump and comprising an outlet orifice for dispensing the fluid, said nozzle being provided with an expulsion channel and with a closure member disposed in fixed manner in the expulsion channel upstream from said outlet orifice, said closure member comprising a fixed portion fixed relative to the outlet orifice and an elastically-deformable portion supporting a closure element which, at rest, closes off the outlet orifice and which, when the elastically-deformable portion deforms, opens the outlet orifice.

6. A dispenser according to claim 5, wherein the pump comprises
a pumping chamber which has an inlet valve and is actuated by said pumping button, which is provided along one of said major surfaces, wherein said pumping button is formed as a part of one of said major surfaces and serves as a wall of said pumping chamber, and depression of the pumping button to its displaced position causes liquid contained in said pumping chamber to be expelled and return of said button to its first normal position causes liquid to enter said pumping chamber via said inlet valve, the dispensing nozzle being in fluid communication with the pumping chamber.

7. A dispenser formed according to any preceding claim, further comprising a cover that engages with the body of the dispenser so as to cover and protect at least the outlet orifice from the external environment, the cover comprising a closure element that, when the cover is fitted in the closed position, sealing engages with the outlet orifice and/or with a surface surrounding the outlet orifice.

8. A pump for dispensing a fluid from a reservoir, comprising a pump chamber comprising a bottom plate, inlet and outlet ports, an actuator that forms at least part of at least one of the pump chamber walls, and a deformable resistance device within the pump chamber, interposed between the bottom plate and the actuator, such that the deformable resistance device does not interfere significantly with the initial deformation of the actuator during actuation but resists the final deformation of the actuator, the deformation of the deformable resistance device not occurring until a threshold force is overcome, thereby providing a feedback signal to the user in order to determine complete actuation.

9. A fluid dispenser comprising a reservoir containing the fluid substance to be dispensed, and an outlet orifice, said reservoir including at least one actuating wall or wall portion that can be deformed by applying a pressing force so as to reduce the internal volume of the reservoir and thus exert pressure on the fluid substance so as to deliver it through the outlet orifice, said at least one actuating wall having a predetermined threshold of resistance to deformation that must be overcome in order to deform it; wherein the predetermined threshold of resistance to deformation of the actuating wall is generated by threshold resistance means positioned immediately under said actuating wall or wall portion, said threshold resistance means exerting substantially no pressure on the fluid contained within the reservoir once the predetermined threshold of resistance to deformation is overcome.

10. A dispenser according to any preceding claims, wherein the outlet defines a fluid delivery axis, and wherein the pump is actuated by a force directly applied to the dispenser at an angle of at least 60° to the fluid delivery axis.

11. A fluid dispenser comprising:
a) a body comprising an upper half comprising a major surface and a lower half comprising a major surface, the major part of said body being substantially rigid, the two major surfaces being opposite one another and having peripheral edges which are interconnected to define therebetween a substantially closed reservoir for containing a liquid to be dispensed therein, the peripheral edges of the two halves comprising connection means to enable fluid tight sealing of the two halves, wherein the peripheral edges of the two halves define a fluid outlet opening when interconnected; and
b) a bridge piece defining at least part of a fluid inlet channel and interposed between the upper and lower halves, the bridge piece further comprising a fluid outlet channel body that emerges from the fluid dispenser via the fluid outlet opening defined by the upper and lower halves, the fluid outlet channel body comprising sealing means that engages the edges of the fluid outlet opening in a fluid-tight seal.

12. A fluid dispenser comprising:
a) a body comprising an upper half comprising a major surface and a lower half comprising a major surface, the major part of said body being substantially rigid, the two major surfaces being opposite one another and having peripheral edges which are interconnected to define therebetween a substantially closed reservoir for containing a liquid to be dispensed therein, the peripheral edges of the two halves comprising connection means to enable fluid tight sealing of the two halves, wherein the peripheral edges of the two halves define a fluid outlet opening when interconnected, wherein one of the said halves is formed from a material which is substantially absorbent to laser radiation of a wavelength capable of welding the two halves together, and the other of the said halves is substantially transmissive to the said radiation; and
b) a bridge piece comprising pumping means and defining at least part of a fluid dispensing channel that sits between the upper and lower halves, the bridge piece further defining a fluid outlet channel body that emerges from the fluid dispenser via the fluid outlet opening defined by the upper and lower halves.

13. A fluid dispenser comprising:
a) an upper dispenser half comprising a major surface, the major part of said upper dispenser half being substantially rigid, the peripheral edges of said upper half extending in a generally perpendicular direction from the plane of the major surface, said upper half further comprising a pumping button formed as a part of said major surface and serving as a wall of a pumping chamber;
b) a bridge piece comprising a pump chamber bottom plate, said pump chamber bottom plate comprising an inlet port and an outlet port in fluid communication with the pumping chamber, wherein the pump chamber bottom plate sealingly engages with the pumping button of the upper dispenser half so as to form a sealed pumping chamber;
c) an inlet valve in fluid communication with the inlet port;
d) an outlet valve in fluid communication with the outlet port; and
e) a lower dispenser half comprising a flexible material sealingly bonded to the perpendicular peripheral edges of the rigid upper dispenser half so as to define a flexible reservoir wall and such that the return of the pumping button following dispensing causes fluid to be taken up into the pumping chamber via the inlet valve and inlet port generating a reduced pressure in the reservoir resulting in deformation of the flexible lower dispenser half to adapt to the reduced volume therein.

14. A dispenser according to any one of claims 3, 4, 5, 6 and 13, wherein said button is fully integral with the major surface of which it is part.

15. A dispenser according to any one of claims 3, 4, 5, 6, and 13, wherein said button is semi-integral with the major surface of which it is part.

16. A dispenser according to any one of claims 3, 4, 5, 6 and 13, wherein said button is non-integral with the major surface of which it is part.

17. A liquid dispenser according to any preceding claim, wherein the reservoir contains a liquid in the form of a medicine.

18. A dispenser according to claim 17, wherein the medicine is a composition for treatment of the human respiratory tract.

19. A dispenser according to claim 18, wherein the composition is adapted for nasal treatment

20. A dispenser according to any one of claims 17 to 19, wherein the liquid is preservative-free.

21. A bridge piece for a modular fluid dispenser and pump, said bridge piece being of unitary construction and comprising:
a) an inlet channel wall defining at least part of an inlet fluid channel;
b) a pump chamber bottom surface defining at least one wall of a pump chamber;
c) an inlet port communicating the inlet fluid channel with the pump chamber;
d) an outlet port in fluid communication with the pump chamber; and
e) a fluid outlet channel body in fluid communication with the outlet port, said fluid outlet channel body defining a fluid outlet channel therein.
